# FASCICULE DE BREVET EUROPEEN

(11) **EP 0 278 829 B1**
(45) Date de publication et mention de la délivrance du brevet: **31.03.1993**
(21) Numéro de dépôt: 88400155.3
(22) Date de dépôt: 25.01.1988
(51) Int. Cl.: A61N 5/10, G01B 5/04, F16C 1/12

(54) **Dispositif d'entrainement et de positionnement d'un porte-sources dans un applicateur utilisé en curiethérapie**
Einrichtung zum Antrieb und zur Einstellung eines Quellenhalters in einer in Radioaktivtherapie verwendeten Einbringungsvorrichtung
Device for driving and positioning a source holder in an applicator used in radioactive therapy

(30) Priorité: 28.01.1987 FR 8700992
(43) Date de publication de la demande: 17.08.1988
(73) Titulaire: CIS BIO INTERNATIONAL, F-91000 Saclay (FR)
(72) Inventeur: Rague, Bruno, F-91440 Bures Sur Yvette (FR); Olombel, André, F-91740 Briis Sous Forges (FR)
(74) Mandataire: Mongrédien, André

(56) Documents cités:
- EP-A- 0 158 630
- EP-A- 0 189 148
- FR-A- 2 474 374
- US-A- 3 669 093

## Description

La présente invention se rapporte au domaine de la curiethérapie et a plus spécialement pour objet un dispositif servant à l'introduction et au positionnement d'un porte-sources dans un tube applicateur.

Il est connu, pour soigner certaines maladies telles que les cancers du sein par exemple, d'introduire à l'intérieur de l'organe malade un ou plusieurs tubes de faible diamètre appelés "tubes applicateurs" ou "applicateurs" dans lesquels on introduit une ou plusieurs sources radioactives qu'on laisse agir pendant une durée variable, généralement de l'ordre de un à deux jours. Il y a le plus souvent une vingtaine d'applicateurs, leur nombre étant généralement compris entre 5 et 30. Les éléments les plus couramment utilisés pour réaliser les sources sont l'iridium, le cobalt, le césium et le californium. Ces sources peuvent être contenues à l'intérieur d'un tube appelé "porte-sources" et c'est ce porte-sources qui est introduit dans l'applicateur. Elles peuvent se présenter soit sous forme de grains de quelques millimètres de longueur, soit sous forme de fils de plus grande longueur. Le porte-sources peut donc contenir soit un fil unique, soit une pluralité de grains disposés les uns à la suite des autres et séparés par des entretoises. La longueur active, c'est-à-dire la longueur occupée par les sources, est au plus égale à 150 mm. Le porte-sources est monté à la partie antérieure d'un câble mobile le long d'un tube-guide qui débouche dans l'applicateur.

Les problèmes qui se posent sont non seulement celui de l'entraînement du cable entre une position initiale, dite "position de stockage", dans laquelle le porte-sources est par exemple à l'intérieur d'un bloc de plomb, jusqu'à une position finale ou position de traitement dans laquelle le porte-sources se trouve dans l'applicateur, mais également celui du positionnement du porte-sources car celui-ci doit se trouver à un endroit très précis à l'intérieur de l'applicateur, et également le problème de la détection d'obstacle. En effet, il importe de détecter tout obstacle rencontré par le câble avant qu'il atteigne la position finale et de déterminer l'emplacement de cet obstacle afin de pouvoir intervenir rapidement.

Plusieurs solutions ont été proposées à ce jour.

Le document FR-A-2 033 653 décrit un dispositif utilisé notamment en plésiocuriethérapie dans lequel le tube porte-sources est entraîné par un câble métallique. Le contrôle de la position de l'extrémité postérieure de ce câble (c'est-à-dire l'extrémité opposée à celle qui porte les sources) se fait à l'aide de deux détecteurs de fin de course qui détectent le passage de cette extrémité. Il n'est pas prévu de détection d'obstacle dans ce dispositif, ce qui fait que le câble risque de se bloquer avant que son extrémité postérieure ne passe le détecteur de fin de course correspondant à la position finale, donc que les sources ne se trouvent pas dans l'applicateur.

Le document FR-A-2 536 531 a pour objet un procédé de contrôle de positionnement d'un porte-sources dans un dispositif de ce genre. Le contrôle se fait par analyse du courant d'induit du moteur entraînant le câble : la présence d'un obstacle sur le parcours fait que le courant d'induit du moteur change, ce qui permet de détecter la présence de cet obstacle. Une variation d'intensité de ce courant représente une augmentation du couple résistant et la détection de cette variation permet de définir un temps de déplacement du câble sur lequel est monté le porte-sources. Ce temps de déplacement est comparé à un temps de référence, ce qui permet de déterminer si le câble occupe une position extrême de son parcours ou une position intermédiaire. Si un tel procédé permet de détecter la présence d'un obstacle, il n'est pas possible de déterminer la position exacte de celui-ci, car il peut y avoir un certain glissement entre le tube et le galet du moteur d'entraînement et on ne connaît pas la longueur exacte de câble qui s'est déroulée. Pour la même raison on n'est pas sûr non plus que le porte-sources soit bien à sa place dans l'applicateur lorsque le câble est dans sa position finale.

Le document EP-A-0 152 124 décrit encore un dispositif d'entraînement et de positionnement d'un porte-sources dans un applicateur utilisé en curiethérapie. Dans ce dispositif, le porte-sources est actionné par un câble métallique dont l'extrémité opposée aux sources est enroulée sur une rainure en forme de spirale prévue sur une face d'une roue entraînée par une crémaillère. Le positionnement du porte-sources dans l'applicateur se fait à l'aide d'un système pneumatique. On envoie de l'air comprimé à travers une ouverture située à une extrémité du tube-guide dans lequel circule le câble et l'air sort par une ouverture située à l'extrémité de ce tube-guide la plus proche de l'applicateur. Le câble porte une tête qui vient en contact avec une butée lorsque le porte-sources est à sa place dans l'applicateur, ce qui empêche la sortie de l'air. Un appareil de contrôle détecte l'augmentation de pression qui en résulte à l'intérieur de la gaine et commande l'arrêt de l'entraînement du câble.

Par contre, il n'est pas prévu de système de détection d'obstacle dans ce dispositif. Il en résulte que, si le câble est arrêté par un obstacle avant sa position finale, l'air continuera à sortir par le deuxième orifice du tube-guide et le câble continuera à se dérouler. On ne peut donc ni savoir qu'il y a un obstacle, ni détecter la position de celui-ci.

On connaît également par le document EP-A-0 158 630 un autre dispositif d'introduction de sources radioactives. Il comprend un câble portant des sources radioactives à une de ses extrémités et étant entraîné en translation par un moteur et une roue autour de laquelle il s'enroule. Il possède un tube-guide pour le câble. Avec ce dispositif, il est possible de savoir si le câble qui porte les sources est à l'extrémité postérieure de l'applicateur.

Avec ce système il est possible de savoir si la source a atteint soit l' extrémité du tube-guide, soit un obstacle, en comparant le nombre de pas effectivement faits par le moteur pas à pas avec le nombre de pas qu'il aurait dû faire.

La présente invention a pour but de remédier à ces inconvénients en proposant un dispositif d'entraînement et de positionnement d'un porte-sources dans un applicateur utilisé en curiethérapie qui permet non seulement de déplacer aisément le porte-sources mais encore de le positionner de façon très précise dans l'applicateur, et qui permet de détecter rapidement un obstacle et de déterminer facilement et avec précision l'emplacement de celui-ci.

Plus précisément, la présente invention a pour objet un dispositif d'entraînement et de positionnement d'au moins un porte-sources dans un tube applicateur utilisé en curiethérapie, ce dernier ayant une première extrémité et une deuxième extrémité, cette deuxième étant ouverte, ce dispositif comprenant de manière connue :
- un tube-guide ayant une extrémité connectée à ladite deuxième extrémité de l'applicateur ;
- un câble se déplaçant longitudinalement le long de ce tube-guide et de l'applicateur entre une première position et une deuxième position, ce câble ayant une première extrémité au voisinage de laquelle ledit porte-sources monté et une deuxième extrémité ; et
- des moyens d'entraînement de ce câble le long du tube-guide et de l'applicateur au moyen d'un moteur.

Selon l'invention, les moyens d'entraînement comprennent :
- un galet moteur pouvant être mise en contact tangentiel avec glissement avec le câble et entraîné par le moteur ; et
- un codeur équipé d'un galet pouvant être mis en contact tangentiel sans glissement avec le câble, ce codeur étant apte à détecter une interruption de la rotation de ce galet.

L'expression "dispositif d'entraînement et de positionnement d'au moins un porte-sources dans un tube applicateur" signifie que l'invention s'applique quel que soit le nombre de tubes applicateurs. Elle est utilisable pour placer un seul porte-sources dans un applicateur, mais, en curiethérapie, on utilise généralement plusieurs porte-sources par applicateur et chaque porte-sources contient une ou plusieurs sources.

De préférence, le codeur utilisé est un codeur incrémental, c'est-à-dire qu'il envoie un signal chaque fois que le galet s'est déplacé en rotation d'un angle donné, c'est-à-dire pratiquement chaque fois que le câble s'est déplacé d'une longueur déterminée.

Avantageusement, on peut prévoir un détecteur devant lequel peut passer le porte-sources lorsqu'il est monté sur le câble au cours du mouvement de celui-ci afin de compter les sources. Ce détecteur permet donc non seulement de compter les sources montées sur un cable donné, mais également de vérifier que le nombre de sources montées sur ce câble est correct.

Selon un autre aspect de l'invention, le dispositif comporte en outre un premier détecteur de fin de course au voisinage de la deuxième extrémité du câble lorsque celui-ci est à sa première position.

Avantageusement, on peut prévoir en outre un deuxième détecteur de fin de course placé de manière à détecter le passage de la deuxième extrémité du câble si celui-ci va au-delà de sa deuxième position.

Enfin, on peut prévoir également un deuxième tube-guide dans lequel peut se déplacer au moins une partie du câble, ce deuxième tube-guide ayant une extrémité fermée par une butée, la deuxième extrémité du câble venant en contact avec cette butée si le câble vient en deça de sa première position.

L'invention apparaîtra mieux à la lecture de la description qui va suivre, donnée à titre d'exemple purement illustratif et nullement limitatif, en référence aux dessins annexés, dans lesquels :
- la figure 1 est une vue schématique en perspective illustrant le principe général de la curiethérapie,
- la figure 2 est une vue schématique en coupe illustrant les principaux éléments du dispositif objet de l'invention,
- la figure 3 est une vue schématique en perspective montrant à plus grande échelle le moteur d'entraînement du câble et le codeur,
- la figure 4 est une vue schématique montrant en coupe de manière plus détaillée les connexions du tube contenant plusieurs tubes-guides, d'une part avec la machine et, d'autre part, avec la ceinture portée par la patiente, et
- la figure 5 est une vue schématique montrant comment on peut disposer plusieurs dispositifs conformes à l'invention à l'intérieur d'une machine.

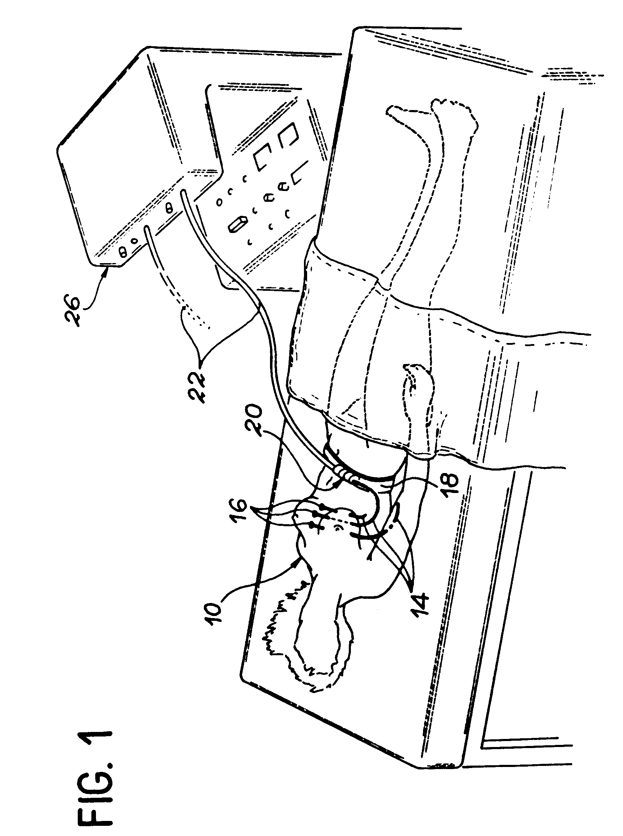

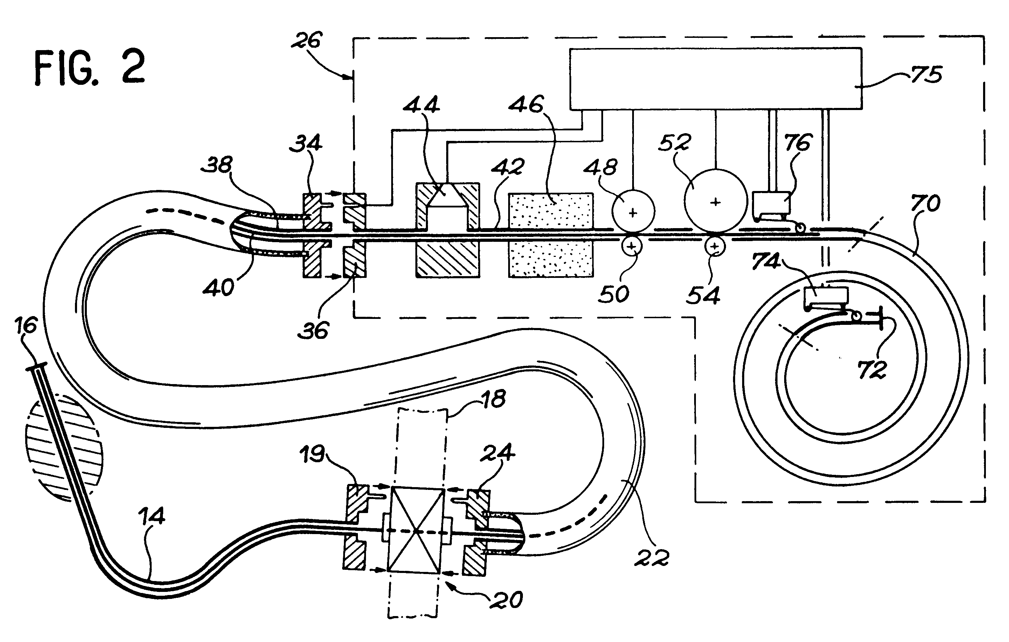

Sur la figure 1, on voit une malade 10 qui est atteinte d'un cancer du sein et se trouve allongée sur une table de traitement 12. Le dispositif de traitement comporte un certain nombre d'applicateurs 14 qui sont des tubes de petit diamètre dont au moins une partie a été introduite à l'intérieur de l'organe malade. En général, il y a une vingtaine de tubes applicateurs. Ces derniers ont une première extrémité fermée par une butée 16 tandis que leur autre extrémité est reliée à un système de connexion 20 monté sur une ceintue 18 portée par la patiente. Un tube 22 de diamètre relativement important porte à l'une de ses extrémités un connecteur 24 (figure 2) qui lui permet d'être relié au dispositif 20 tandis que son autre extrémité est reliée à une machine 26 dans laquelle se trouvent les dispositifs d'entraînement des câbles sur lesquels sont montés les porte-sources.

Le principe du traitement par curiethérapie est le suivant :
Dans un premier temps, le tube 22 est relié à la machine 26 et déconnecté du dispositif 20. Un certain nombre de câbles placés chacun dans un tube-guide se trouve à l'intérieur du tube 22 et leur déplacement est commandé par un dispositif situé à l'intérieur de la machine. Les porte-sources destinés à être placés à l'intérieur des applicateurs 14 se trouvent dans un conteneur (non représenté sur la figure 1).

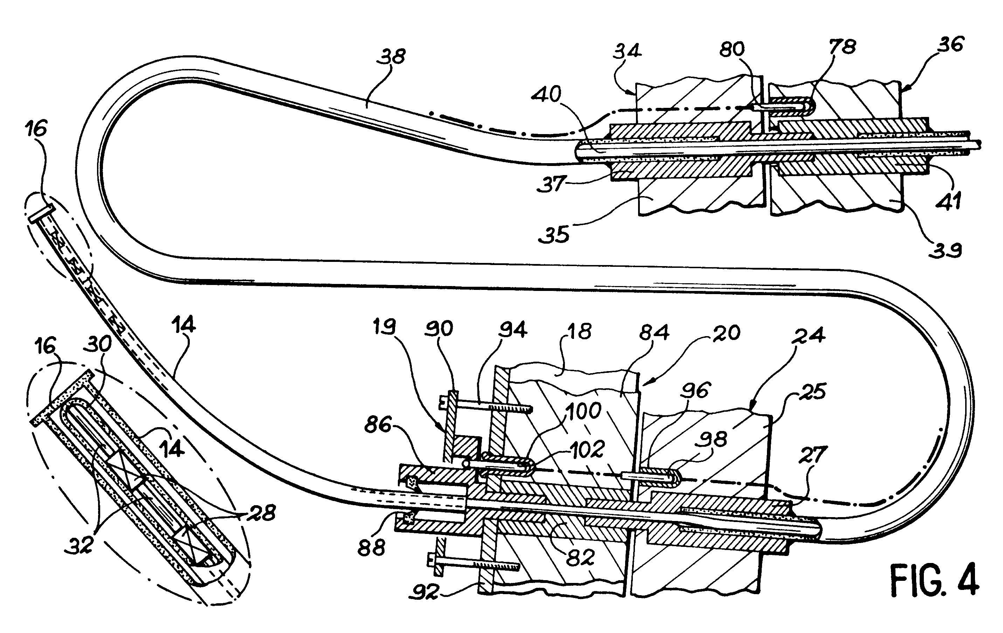

Le détail agrandi de la figure 4 montre un tel porte-sources. Sur cette figure, on voit les sources 28 placées les unes derrière les autres à l'intérieur du tube porte-sources 30. Les sources sont séparées les unes des autres par des entretoises 32. On place un tel tube 30 à l'extrémité de chacun des câbles qui circulent à l'intérieur des tubes-guides contenus dans le tube de grand diamètre 22.

Les tubes 30 étant initialement placés dans un conteneur, on connecte d'abord le tube 22 avec ce conteneur et on manoeuvre les câbles afin que chacun d'eux puisse aller chercher un tube 30 dans le conteneur. Le montage des tubes 30 sur les câbles se fait à l'aide de moyens d'accrochage qui sont connus en soi et ne seront pas décrits ici. On manoeuvre ensuite les câbles le long de leur tube-guide afin de les amener en une position telle que les sources soient placées à l'intérieur d'un bloc de protection, par exemple en plomb, prévu à l'intérieur de la machine 26. On déconnecte le tube 22 du conteneur et on le connecte au dispositif 20. On déplace ensuite les câbles en sens inverse jusqu'à ce que les tubes 30 arrivent dans les applicateurs 14 et que leurs extrémités soient en contact avec les butées 16 de ces applicateurs.

On laisse ensuite agir les sources pendant le temps nécessaire, qui est de l'ordre de 1 à 2 jours. Une fois le traitement terminé, on manoeuvre à nouveau les câbles pour les ramener à leur position initiale ou position de stockage dans laquelle les sources sont à l'intérieur du bloc de plomb. Le tube 22 est ensuite déconnecté du dispositif 20 et on retire les applicateurs 14.

Il est à noter que les éléments radioactifs utilisés en curiethérapie ont généralement des périodes courtes (par exemple, 74 jours pour l'iridium). En conséquence, les sources ne sont pas réutilisées pour un deuxième traitement et on les envoie après utilisation dans une installation de stockage et/ou de traitement de déchets radioactifs.

La vue en coupe de la figure 2 montre de manière plus détaillée le dispositif d'entraînement et de positionnement conforme à l'invention qui permet de manoeuvrer le câble sur lequel est monté le porte-sources. On retrouve sur cette figure le tube applicateur 14 qui est relié au dispositif 20 de la ceinture 18 à l'aide d'un connecteur 19. On voit également le tube 22 dont une extrémité peut être reliée au dispositif 20 par un connecteur 24 et dont l'autre extrémité peut être reliée à un connecteur 36 prévu sur une paroi de la machine 26 par un connecteur 34. On a également représenté, à l'intérieur du tube 22, un tube-guide 38 à l'intérieur duquel peut se déplacer le câble 40 à une extrémité duquel est monté le porte-sources. Sur la figure 2, on a représenté un seul applicateur 14 et un seul câble 40 dans son tube-guide 38, mais, en réalité, il y a plusieurs tubes-guides 38 à l'intérieur du tube 22 et plusieurs applicateurs. Les connecteurs tels que 19, 24, 34 et 36 sont prévus pour le passage de plusieurs câbles 40, par exemple dix câbles.

On voit également sur la figure 2 un deuxième tube-guide 42 placé à l'intérieur de la machine et dans lequel le câble 40 peut se déplacer. Un détecteur 44 permet de compter le nombre de sources se trouvant à l'extrémité du câble 40 lorsque celui-ci est ramené à l'intérieur de la machine 26. Il est à noter qu'il y a autant de détecteurs 44 que de câbles 40. Le rôle de ces détecteurs est non seulement de compter les sources se trouvant sur un porte-sources donné, mais également de vérifier que le nombre de sources se trouvant sur ce porte-sources est bien celui qui était prévu.

Toujours à l'intérieur de la machine 26 se trouve un bloc 46 qui peut être un bloc de plomb et qui est traversé par le tube-guide 42. La première position du câble 40 ou position de stockage est celle dans laquelle le câble est suffisamment rentré à l'intérieur de la machine pour que les sources se trouvent à l'intérieur de ce bloc. On assure ainsi une protection et on évite l'irradiation du personnel en attendant que les connexions soient effectuées afin de pouvoir manoeuvrer le câble 40 en sens inverse pour amener les sources dans l'applicateur 14.

On voit encore sur la figure 2 les moyens d'entraînement du câble 40 le long des tubes-guides 38 et 42. Selon l'invention, ces moyens d'entraînement comprennent un premier galet ou galet moteur 48 actionné par un moteur non représenté sur la figure 2. Ce galet 48 est en contact tangentiel avec le câble et il peut glisser légèrement par rapport à celui-ci. Un contre-galet 50 est prévu en face du galet 48.

Les moyens d'entraînement comprennent en outre un deuxième galet 52 qui est également en contact tangentiel avec le câble 40, mais ne peut pas glisser par rapport à celui-ci. Un contre-galet 54 est prévu en face du galet 52. Ce dernier est relié à un codeur (non représenté sur la figure 2).

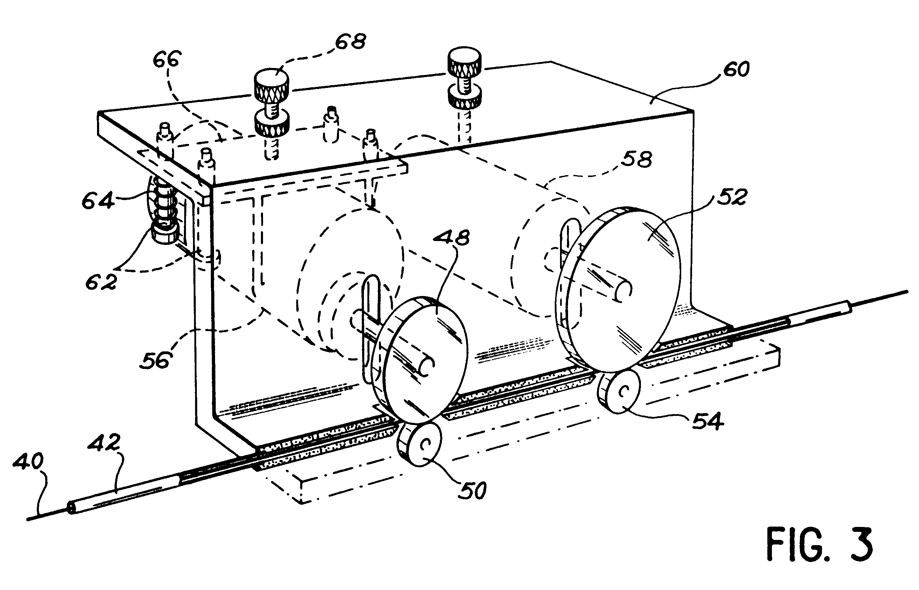

Les moyens d'entraînement sont représentés plus en détail sur la vue en perspective de la figure 3.

On voit sur cette figure que le moteur 56 et le codeur 58 sont montés sur un support 60. Un dispositif est prévu pour régler la position en hauteur du moteur 56 et du codeur 58 et donc la force d'application des galets 48 et 52 sur le câble.

C'est par un réglage convenable de ces forces d'application qu'on peut faire en sorte que le galet 48 soit en contact tangentiel avec glissement avec le câble 40 et que le galet 52 soit en contact tangentiel avec le cable 40 sans pouvoir glisser par rapport à celui-ci. Pour cela, on a prévu, montées sur le support 60, quatre colonnes de guidage verticales 62. Un ressort 64 est placé autour de chacune des colonnes entre la partie inférieure de celles-ci et une plaque 66 dont la position peut être réglée à l'aide d'une vis 68. La plaque 66 est liée rigidement au moteur 56. C'est la vis 68 qui permet de déplacer la plaque 66 à l'encontre de l'action exercée par les ressorts 64 donc d'abaisser plus ou moins le moteur 56 et d'appliquer plus ou moins le galet 48 sur le câble 40. Un dispositif semblable est utilisé pour le codeur 58. Bien entendu, dans le mode de réalisation représenté ici, les contre-galets 50 et 54 sont fixes par rapport au support 60.

Il est bien entendu qu'il ne s'agit là que d'un exemple d'un système apte à régler la force d'application des galets 48 et 52 sur le câble 40 et qu'on ne sortirait pas du cadre de l'invention en utilisant un système équivalent. L'essentiel est que le galet moteur 48 puisse glisser par rapport au câble et que le galet 52 ne le puisse pas.

Si l'on se reporte à nouveau à la figure 2, on voit que les détecteurs 44 et le bloc de plomb 46 se trouvent, par rapport au câble 40 et aux tubes-guides 38 ou 42 qui le contiennent, entre les galets 48 et 52 et le connecteur 36 permettant le raccordement du tube extérieur 22.

On voit encore sur la figure 2 un troisième tube-guide 70 placé du côté opposé aux détecteurs 44 et au bloc 46 par rapport aux galets 48 et 52. La longueur du tube 70 est suffisante pour permettre à la deuxième extrémité du câble, c'est-à-dire celle qui est opposée au porte-sources, de se déplacer lorsque le câble est ramené à la position de stockage dans laquelle les sources se trouvent à l'intérieur du bloc 46. Le tube 70 est fermé à son extrémité opposée aux galets 48 et 52 par une butée 72. De plus, deux détecteurs de fin de course 74 et 76 sont placés respectivement à proximité de la butée 72 et au voisinage du galet 52. Leur emplacement est tel que la distance entre ces deux détecteurs, comptée le long du tube-guide 70, est légèrement supérieure à la distance parcourue par le câble lorsque celui-ci va de la position de stockage à la position de traitement dans laquelle le porte-sources est en place dans l'applicateur. Le rôle de ces deux détecteurs de fin de course et de la butée 72 sera décrit plus loin dans la présente description.

Enfin, un automate programmable 75 prévu à l'intérieur de la machine 26 permet de commander tous les appareils, c'est-à-dire essentiellement les détecteurs 44, le moteur 56, le codeur 58 et les détecteurs 76 et 74.

La vue en coupe de la figure 4 montre de manière plus détaillée les connecteurs permettant de relier le tube 22 à la machine 26 et au dispositif 20 équipant la ceinture 18 de la malade, ainsi que le système de connexion permettant de fixer les applicateurs 14 sur le dispositif 20.

On voit sur la figure 4 que le connecteur 36 de la machine 26 se compose d'une plaque 39 dans laquelle sont prévus des trous permettant de loger des fiches femelles 41. De manière similaire, le connecteur 34 équipant l'extrémité du tube 22 du côté de la machine se compose d'une plaque 35 dans laquelle ont été ménagées des ouvertures permettant de loger des fiches mâles 37. Chacune des fiches mâles 37 et femelles 41 est percée de part en part par un canal cylindrique permettant le passage du tube-guide 38 contenant le câble 40.

On voit encore qu'on a prévu une fiche femelle 78 sur la plaque 39 et une fiche mâle 80 montée sur la plaque 35 afin d'assurer la continuité électrique de l'ensemble.

A l'autre extrémité du tube 22 (non représenté sur la figure 4), le connecteur 24 se compose d'une plaque 25 dans laquelle ont été ménagés plusieurs orifices pour le logement de fiches mâles 27. Chaque fiche mâle 27 est prévue pour être introduite dans un raccord 82, lui-même monté sur un support 84 prévu sur la ceinture 18 de la patiente. Une fiche mâle 86 est montée à l'extrémité de l'applicateur 14 opposée à la butée 16, c'est-à-dire son extrémité opposée à celle où se trouvent les sources radioactives en position de traitement. Un joint à lèvres 88 assure l'étanchéité à l'endroit où l'applicateur 14 pénètre à l'intérieur de la fiche 86. Celle-ci est prévue pour coopérer avec le raccord 82. Ce dernier, ainsi que les fiches 27 et 86 sont prévus avec un canal central permettant le passage du câble 40.

Le maintien en position des fiches 86 est assuré par une plaque de verrouillage 90 qui est fixée sur une plaque de maintien 92 à l'aide de vis 94. La plaque 25 est prévue avec une fiche femelle 96 qui peut coopérer avec une fiche male 98 portée par le support 84. De même, du côté de l'applicateur 14, ce dernier est prévu avec une fiche femelle 100 qui peut coopérer avec une fiche mâle 102 montée sur la plaque 90. Les plaques telles que 39, 35 ou 25 peuvent être prévues avec un nombre quelconque d'orifices, par exemple 10, et permettre ainsi le raccordement simultané d'un nombre égal de tube-guides. En ce qui concerne le dispositif de connexion 20, il y a autant de raccords tels que 82 qu'il y a de fiches 27 et de fiches 86 à raccorder.

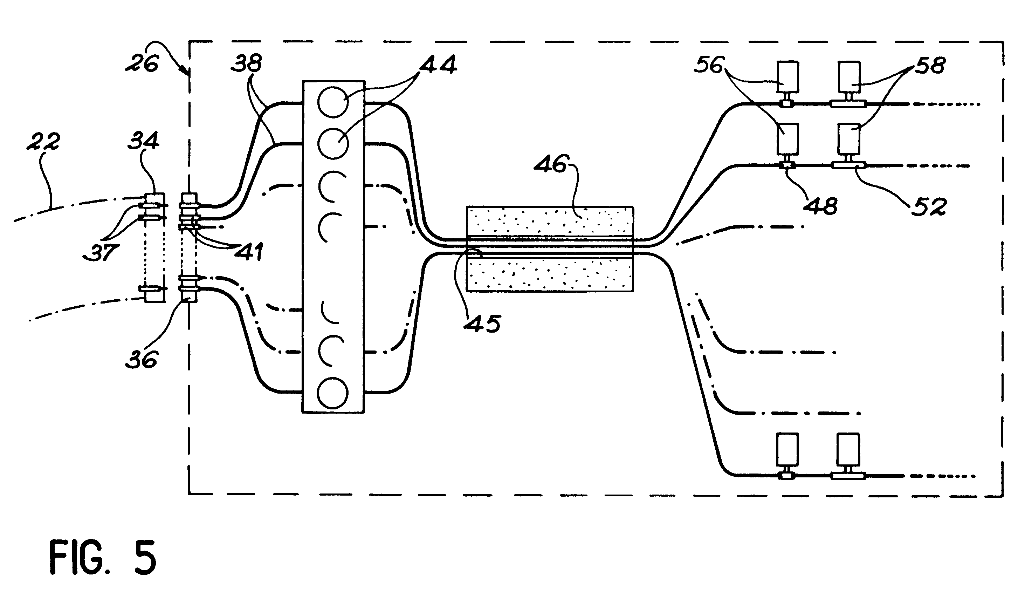

La vue schématique de la figure 5 montre comment on peut installer plusieurs dispositifs d'entraînement selon l'invention à l'intérieur d'une machine donnée.

Dans le cas particulier décrit ici, on a représenté un groupe de dix dispositifs d'entraînement, mais on peut prévoir, dans une seule machine, deux groupes comprenant chacun dix dispositifs semblables. Bien entendu, on ne sortirait pas du cadre de l'invention en modifiant le nombre de groupes et/ou le nombre de dispositifs dans un groupe donné.

On voit sur cette figure les connecteurs 34 monté à l'extrémité du tube 22 et 36 monté sur une paroi de la machine. Du connecteur 36 partent plusieurs tubes-guides 38 (10 dans l'exemple représenté ici) dont chacun passe devant un détecteur 44. L'ensemble des tubes-guides passe ensuite dans le canal central 45 du bloc de plomb 46. Il est à remarquer que, s'il est nécessaire d'avoir un détecteur 44 pour chaque tube 38 afin de compter les sources se trouvant dans un porte-sources donné, il n'est pas nécessaire d'avoir autant de blocs de protection 46 que de tubes 38. On simplifie la construction et on diminue le poids de la machine en utilisant un seul bloc muni d'un canal de dimensions suffisantes pour permettre le passage de tous les tubes-guides. Du côté du bloc 46 opposé au détecteur 44, le faisceau de tubes-guides s'écarte afin que chaque câble puisse passer devant un moteur 56 et un codeur 58. En effet, on comprend d'après ce qui précède que les galets équipant respectivement un moteur et un codeur n'agissent que sur un seul câble et qu'il faut autant d'ensembles moteurs-codeurs tels que celui représenté à la figure 3 qu'il y a de câbles. De même, il y a autant de tubes-guides 70, tels que celui représenté à la figure 2, et éventuellement de détecteurs 74 et 76 montés sur un tel tube-guide, qu'il y a de câbles à l'intérieur de la machine.

Le fonctionnement du dispositif d'entraînement conforme à l'invention est le suivant :
Le galet moteur 48 monté sur le moteur 56 entraîne le câble lorsque le moteur est sous tension. Ceci permet d'avancer le câble le long des tubes-guides tels que 40 et 42 et d'amener le cable à toutes les positions désirées : position d'accrochage des porte-sources se trouvant dans un conteneur, position de stockage dans laquelle les porte-sources sont ramenés à l'intérieur de la machine, à l'intérieur du bloc 46, et position de traitement dans laquelle les porte-sources se trouvent dans l'applicateur 14. Le galet 52 équipant le codeur est en contact tangentiel sans glissement avec le câble 40. Ceci fait que, lorsque le câble est en mouvement, le galet 52 est entraîné en rotation. Si le câble rencontre un obstacle sur son parcours, il s'immobilise aussitôt. Comme le galet 52 ne peut pas gliser par rapport au câble, sa rotation s'arrête également aussitôt. Cependant, comme le galet moteur 48 peut glisser par rapport au câble, il continue à tourner. Comme le codeur 58 est apte à envoyer des signaux fonction de la position angulaire du galet 52, on peut détecter la longueur de câble qui s'est déroulée depuis une position de départ prédéterminée lorsque la rotation du galet 52 est interrompue. On peut donc savoir si l'obstacle rencontré par le câble est la butée 16 se trouvant à l'extrémité de l'applicateur 14 ou un obstacle parasite se trouvant ailleurs le long du parcours. Ainsi, connaissant la longueur de câble qui s'est déroulée, on peut déterminer avec une bonne précision l'emplacement de l'obstacle rencontré par l'extrémié du câble.

Dans le mode de réalisation préféré, le codeur 58 est un codeur incrémental, c'est-à-dire qu'il envoie un signal chaque fois que le galet 52 a tourné d'un angle déterminé, c'est-à-dire pratiquement chaque fois que le câble 40 s'est déplacé d'une longueur donnée. Si le câble 40 cesse de se déplacer, le codeur cesse d'envoyer des signaux et cet état est détecté par l'automate programmable 75 qui constate que le galet 52 ne tourne plus. Comme le galet 48 peut glisser par rapport au câble, il continue à tourner. L'automate programmable commande alors l'arrêt du moteur. D'autre part, si l'obstacle rencontré n'est pas la butée 16 se trouvant à l'extrémité de l'applicateur 14, l'automate commande le retour du porte-sources dans le bloc 46 afin d'éviter l'irradiation du personnel. On peut alors réinjecter le même porte-sources dans son tube-guide car il se peut qu'il n'y ait pas réellement d'obstacle et que le porte-sources ou le câble ait été bloqué par exemple contre la paroi interne du tube-guide. Si le dispositif détecte à nouveau un obstacle, un opérateur interviendra pour éliminer celui-ci.

Si la longueur de câble qui s'est déroulée depuis une position de départ donnée jusqu'à interruption de la rotation du galet 52 correspond à une position dans laquelle le tube porte-sources 30 est à l'extrémité de l'applicateur 14, on saura que l'obstacle n'est autre que la butée 16 située à l'extrémité de l'applicateur et on sera sûr que les sources radioactives sont en bonne position à l'intérieur de l'applicateur.

Il est à noter que le câble peut subir certaines ondulations et que la longueur de câble ayant défilé devant le galet 52 soit légèrement supérieure à la distance réellement parcourue par le porte-sources. Des essais préalables permettront de déterminer la longueur de câble correspondant à la position dans laquelle le porte-sources est en contact avec la butée 16.

Des sécurités sont prévues pour arrêter le déroulement du cable dans un sens ou dans l'autre afin que le déplacement du câble reste confiné dans des limites prédéterminées. Il pourrait se produire, par exemple, que la butée 16 manque à l'extrémité de l'applicateur 14. C'est pour cela qu'on a prévu le détecteur de fin de course 76. Celui-ci est placé, par rapport aux galets 48 et 52, du côté opposé à l'applicateur 14. De plus, son emplacement est tel que, si l'extrémité antérieure ou première extrémité du câble, c'est-à-dire celle qui porte les sources radioactives, sort de l'applicateur 14 en cas d'abscence accidentelle de la butée 16, la deuxième extrémité ou extrémité postérieure du câble passe au bout d'un temps très court devant le détecteur 76. Celui-ci peut alors envoyer un signal à l'automate 75 et ce dernier commandera l'arrêt du moteur 48 et éventuellement l'envoi d'un signal d'alarme. Il est à noter que le détecteur 76 n'intervient que dans ce cas-là et que, si l'extrémité antérieure du câble, ou plus exactement l'extrémité antérieure du tube porte-sources, vient en contact avec la butée 16, l'autre extrémité du câble n'est pas encore passée devant le détecteur 76.

De même, on a prévu des sécurités pour le mouvement inverse du câble, c'est-à-dire pour assurer l'arrêt de celui-ci lorsqu'on ramène les sources à l'intérieur de la machine 26 afin que ces dernières n'aillent pas au-delà du bloc 46. Il est en effet nécessaire d'arrêter le mouvement du câble à ce niveau-là pour que les sources restent dans le bloc de protection. C'est pour cela qu'on a prévu le détecteur 74. Ainsi, lorsque le câble 40 est ramené à l'intérieur de la machine, ce mouvement continue jusqu'à ce que l'extrémité postérieure du câble passe devant le détecteur 74. A ce moment, celui-ci envoie un signal à l'automate programmable 75 et ce dernier commande l'arrêt du moteur 56. La position du détecteur 74 est telle que l'extrémité postérieure du câble passe devant lui lorsque le tube porte-sources se trouve entièrement à l'intérieur du bloc 46.

Enfin, en cas de défaillance du détecteur 74, on a prévu une butée 72 à l'extrémité correspondante du tube-guide 70. Ainsi, si l'extrémité postérieure du câble passe devant le détecteur 74 sans que celui-ci n'envoie un signal commandant l'arrêt du moteur, le mouvement continuera mais l'extrémité du câble sera bloquée par la butée 72. Comme le câble sera alors brusquement immobilisé, le galet 52 du codeur sera lui aussi immédiatement immobilisé et le codeur enverra un signal identique à celui qui est envoyé lorsque le câble rencontre un obstacle. Comme le système est programmé et que l'on connaît la longueur de câble qui s'est déroulée, on saura que cet arrêt est dû au fait que l'extrémité postérieure du câble est arrivée en contact avec la butée 72. Comme indiqué plus haut, il se peut que, là aussi, le câble subisse quelques ondulations et des essais préalables permettront de déterminer la position du câble pour laquelle sa deuxième extrémité est en contact avec la butée 72.

Le dispositif d'entraînement et de positionnement objet de l'invention présente de nombreux avantages puisqu'il permet de connaître à tout instant la position du câble, de détecter la présence d'un obstacle et de déterminer avec précision l'emplacement de celui-ci. Il permet en outre de s'assurer du positionnement correct des sources dans le tube applicateur. De plus, des sécurités sont prévues pour arrêter le mouvement du câble dans un sens ou dans l'autre en cas de problème, par exemple en cas d'absence de la butée 16 à l'extrémité des applicateurs 14 ou en cas de défaillance du détecteur de fin de course 74.

Enfin, étant donné que le câble peut subir des ondulations, la simple mesure de la longueur de câble qui a défilé ne suffit pas à s'assurer que le porte-sources est en bonne position dans l'applicateur. Le dispositif objet de l'invention permet de s'assurer que le porte-sources est bien à sa place dans l'applicateur puisque le défilement du câble ne s'arrête qu'en cas d'obstacle : si cet obstacle est la butée 16, l'automate ne commande pas le retour du câble et on est sûr du bon positionnement du porte-sources.

Il est encore à noter qu'on change le câble à chaque traitement car on rentre les sources chaque fois que le personnel pénètre dans le local de traitement afin d'éviter l'irradiation. Les mouvements des câbles peuvent être commandés depuis l'extérieur de ce local. Il s'ensuit qu'au cours d'un traitement, chaque câble peut effectuer une cinquantaine d'allers et retours. Le câble est donc fragile et la probabilité de rencontrer un obstacle est accrue : il est donc nécessaire de détecter les obstacles de manière sûre, ce qui est rendu possible par le dispositif objet de l'invention.

Enfin, il est bien entendu que l'invention ne se limite pas au seul mode de réalisation qui vient d'être décrit, mais qu'on peut envisager des variantes sans sortir pour autant du cadre de l'invention. C'est ainsi que l'homme du métier pourra faire varier le nombre et l'emplacement des tubes-guides tels que 40, 42 et 70 dans lesquels circule le câble 40. On pourra utiliser d'autres systèmes de connexion que les connecteurs 34, 36 ou le dispositif 20, ou éventuellement les supprimer si la construction de l'appareil permet de ne pas utiliser de telles connexions. On pourra utiliser n'importe quel type de détecteur pour compter les sources ou éventuellement supprimer ces appareils. De même, les sécurités telles que les détecteurs de fin de course 74 et 76 et la butée 72 ne sont pas obligatoires et pourront éventuellement être remplacées par d'autres systèmes. Quant à la butée 16 placée en bout de l'applicateur 14, elle peut être supprimée et remplacée par une autre butée placée ailleurs le long du trajet du câble. Ce dernier sera alors équipé d'un élément venant en contact avec cette autre butée lorsque les sources sont en bonne place dans l'applicateur. Enfin, si la description a été faite en ne tenant compte que d'un seul câble 40, il est bien entendu qu'on peut placer plusieurs dispositifs selon l'invention dans une machine donnée. Il y aura alors autant de moteurs 56 et de codeurs 58 équipés de galets 48 et 52 qu'il y aura de câbles à manoeuvrer.

## Revendications

1. Dispositif d'entraînement et de positionnement d'au moins un porte-sources dans un tube applicateur (14) utilisé en curiethérapie, ce dernier ayant une première extrémité et une deuxième extrémité, cette deuxième extrémité étant ouverte, ce dispositif comprenant :
- un tube-guide (38) ayant une extrémité connectée à ladite deuxième extrémité de l'applicateur (14) ;
- un câble (40) se déplaçant longitudinalement le long de ce tube-guide (38) et de l'applicateur (14) entre une première position et une deuxième position, ce câble (40) ayant une première extrémité au voisinage de laquelle ledit porte-sources est monté et une deuxième extrémité ; et
- des moyens d'entraînement de ce câble (40) le long du tube-guide (38) et de l'applicateur (14), au moyen d'un moteur (56),
caractérisé en ce que ces moyens d'entraînement comprennent :
- un galet moteur (48) pouvant être mis en contact tangentiel ayec glissement avec le câble (40), et entraîné par le moteur (56); et
- un codeur (58) équipé d'un galet (52) pouvant être mis en contact tangentiel sans glissement avec le câble (40), ce codeur (58) étant apte à détecter une interruption de la rotation de ce galet (52).

2. Dispositif selon la revendication 1, caractérisé en ce que le codeur (58) est un codeur incrémental.

3. Dispositif selon la revendication 1, caractérisé en ce qu'il comporte en outre un détecteur (44) devant lequel peut passer le porte-sources lorsqu'il est monté sur le câble (40), au cours du mouvement de celui-ci afin de compter les sources (28).

4. Dispositif selon la revendication 1, caractérisé en ce qu'il comporte en outre un premier détecteur de fin de course (74) devant lequel se trouve la deuxième extrémité du câble (40) lorsque celui-ci est à sa première position.

5. Dispositif selon la revendication 1, caractérisé en ce qu'il comporte en outre un deuxième détecteur de fin de course (76) placé de manière à détecter le passage de la deuxième extrémité du câble (40) si celui-ci va au-delà de ladite deuxième position.

6. Dispositif selon la revendication 1, caractérisé en ce qu'il comporte en outre un deuxième tube-guide (70) dans lequel au moins une partie du câble (40) peut se déplacer, ce deuxième tube-guide (70) ayant une extrémité fermée par une butée (72), la deuxième extrémité du câble (40) venant en contact avec cette butée (72) si le câble (40) vient en-deçà de ladite première position.

## Claims

1. Device for driving and positioning at least one source holder in an applicator tube (14) used in radiotherapy, the applicator tube having a first end and a second end, the second end being open, said device comprising:
a guide tube (38) having one end connected to said second end of the applicator (14),
a cable (40) displaced longitudinally along said guide tube (38) and said applicator (14) between a first position and a second position, the cable (40) having a first end in the vicinity of which said source holder is mounted, and a second end, and
means for driving the cable (40) along the guide tube (38) and the applicator (14) by means of a motor (56),
characterized in that these drive means include:
a motor-driven roller (48) which can be brought into tangential contact,
with sliding, with the cable (40) and driven by the motor (56) and
an encoder (58) equipped with a roller 952) which can be brought into tangential contact, without sliding, with the cable (40), said encoder being able to detect an interruption in the rotation of this roller (52).

2. Device according to claim 1, characterized in that the encoder (58) is an incremental encoder.

3. Device according to claim 1, characterized in that it further includes a detector (44) past which the source holder moves when it is mounted on the cable (40), during the movement of the cable, in order to count the sources (28).

4. Device according to claim 1, characterized in that it further includes a first end-of-course detector (74) in front of which the second end of the cable (40) is located when the cable is in its first position.

5. Device according to claim 1, characterized in that it further includes a second end-of-course detector (76) located in such a manner as to detect the passage of the second end of the cable (40) if the cable moves beyond said second position.

6. Device according to claim 1, characterized in that it further includes a second guide tube (70) in which at least a portion of the cable (40) can be displaced, this second guide tube (70) having one end closed by a stop (72), the second end of the cable (40) coming into contact with this end (72) if the cable (40) arrives on the near side of this first position.

## Patentansprüche

1. Vorrichtung zum Bewegen und Positionieren von wenigstens einem Quellenhalter in einem Applizierröhrchen oder Applikator (14), verwendet in der Radiumtherapie, die ein erstes und ein zweites Ende aufweist, wobei dieses zweite Ende offen ist, wobei diese Vorrichtung enthält:
- ein Führungsrohr (38), das mit einem Ende an dem genannten zweiten Ende des Applikators (14) befestigt ist;
- ein Kabel 40, das sich in Längsrichtung in diesem Führungsrohr (38) und in dem Applikator (14) bewegt zwischen einer ersten Position und einer zweiten Position, wobei dieses Kabel (40) ein erstes Ende aufweist, in dessen Nähe der genannte Quellenhalter angebracht ist und ein zweites Ende ; und
- Bewegungsmittel für dieses Kabel (40) längs des Führungsrohrs (38) und des Applikators (14) mit Hilfe eines Motors (56),
dadurch **gekennzeichnet,**
daß diese Bewegungsmittel enthalten :
- eine Antriebsrolle (48), die in tangentiellen Gleitkontakt mit dem Kabel (40) gebracht werden kann und angetrieben wird durch den Motor (56) ; und
- einen Codierer (58), an dem eine Rolle (52) angebracht ist, die in tangentiellen Kontakt ohne Gleiten mit dem Kabel (40) gebracht werden kann, wobei dieser Codierer (58) so beschaffen ist, daß er eine Unterbrechung der Rotation dieser Rolle (52) feststellt.

2. Vorrichtung nach Anspruch 1, dadurch gekennzeichnet, daß dieser Codierer (58) ein Inkrementalcodierer ist.

3. Vorrichtung nach Anspruch 1, dadurch gekennzeichnet, daß sie außerdem einen Detektor (44) enthält, an dem sich der Quellenhalter vorbeibewegen kann, wenn er an dem Kabel (40) angebracht ist, während dessen Bewegung, um die Quellen (28) zu zählen.

4. Vorrichtung nach Anspruch 1, dadurch gekennzeichnet, daß sie außerdem einen ersten Endstellungsdetektor (74) enthält, vor dem sich das zweite Ende des Kabels (40) befindet, wenn dieses in seiner ersten Position ist.

5. Vorrichtung nach Anspruch 1, dadurch gekennzeichnet, daß sie außerdem einen zweiten Endstellungsdetektor (76) enthält, der so angeordnet ist, daß er das Vorbeilaufen des zweiten Endes des Kabels (40) feststellt, wenn dieses über die sogenannte zweite Position hinausläuft.

6. Vorrichtung nach Anspruch 1, dadurch gekennzeichnet, daß sie außerdem ein zweites Führungsrohr (70) enthält, in dem sich wenigstens ein Teil des Kabels (40) bewegen kann, wobei dieses zweite Führungsrohr (70) ein durch einen Anschlag (72) verschlossenes Ende aufweist, wobei das zweite Ende des Kabels (40) in Kontakt kommt mit diesem Anschlag (72), wenn das Kabel (40) über die genannte erste Position hinausläuft.
